(19)

Europäisches
Patentamt
European
Patent Office
Office européen
des brevets

(11) **EP 2 250 993 A1**

(12) **EUROPEAN PATENT APPLICATION**
published in accordance with Art. 153(4) EPC

(43) Date of publication:
**17.11.2010 Bulletin 2010/46**

(21) Application number: **09719717.2**

(22) Date of filing: **05.03.2009**

(51) Int Cl.:
*A61K 8/00* (2006.01)     *A61K 8/97* (2006.01)
*A61Q 19/02* (2006.01)     *C12Q 1/02* (2006.01)
*G01N 33/15* (2006.01)     *G01N 33/50* (2006.01)

(86) International application number:
**PCT/JP2009/054199**

(87) International publication number:
**WO 2009/113446 (17.09.2009 Gazette 2009/38)**

(84) Designated Contracting States:
**AT BE BG CH CY CZ DE DK EE ES FI FR GB GR
HR HU IE IS IT LI LT LU LV MC MK MT NL NO PL
PT RO SE SI SK TR**
Designated Extension States:
**AL BA RS**

(30) Priority: **11.03.2008 JP 2008061333**

(71) Applicant: **Shiseido Company, Ltd.
Chuo-ku
Tokyo 104-8010 (JP)**

(72) Inventors:
• **AOKI, Hirofumi
Yokohama-shi
Kanagawa 224-8558 (JP)**
• **ONODERA, Tomoko
Yokohama-shi
Kanagawa 236-8643 (JP)**
• **SATO, Kiyoshi
Yokohama-shi
Kanagawa 224-8558 (JP)**

(74) Representative: **Ulmann, Catherine Claire
Santarelli
14, avenue de la Grande Armée
75017 Paris (FR)**

(54) **SKIN WHITENING METHOD AND SCREENING METHOD FOR FACTORS FOR SKIN WRINKLE FORMATION SUPPRESSION AND/OR REMOVAL**

(57)     A method of skin whitening is provided which includes: selectively activating proliferation and/or differentiation of melanin-containing keratinocytes of the skin.

Fig.1

(a)  (b)  (c)

EP 2 250 993 A1

# Description

[Technical Field]

The present invention relates to a method of skin whitening and a method of screening a skin-spot-formation inhibiting and/or skin-spot removing factor.

[Background Art]

Skin spots occur as one ages, give an impression of aging and occasionally cause a serious skin trouble. A large market has been formed for cosmetics dealing with skin spots. Unlike transient suntans, skin spots occur locally in a sustained manner. Elucidating the mechanism of skin spot formation is required for development of the cosmetics dealing with skin spots. Skin spots can be categorized into several clinical types, among which the present invention relates to solar pigmented macules, typical skin spots which many people suffer as they age. A solar pigmented macule may also be called a senile lentigo (lentigo senilis) or a solar lentigo, all of which terms refer, with slightly different nuances, to an acquired pigmented macule appearing on those areas of seborrheic sites (sites with pilosebaceous glands other than palms and soles) which are chronically exposed to ultraviolet light. Examples include a typical large spot with clear boundaries as often observed on, e.g., the temple of the elderly. Hereinafter, the term "skin spot" means such a solar pigmented macule.

Various methods aimed at solving skin spots have been reported, including: methods for evaluating tendency to skin spot formation based on analysis of spot sites; methods of prevention based on the evaluation; and methods for removing skin spots by controlling factors associated with skin spots. In search of a means to predict a vulnerability to skin spots prior to formation thereof, a report discloses that spotted epidermis and normal epidermis were sampled, and that NT-3, ADAM9 or HB-EGF in each sample was detected as an evaluation index (see Japanese Unexamined Patent Publication No. 2004-205246). Another report suggests inhibition of c-KIT and ET as a countermeasure against skin spots (see Japanese Unexamined Patent Publication No. 2004-83551).

However, there is lingering concern about whether judgment and countermeasure based on such a small number of genes can completely solve the problems. To address this, a report has been made that variations of a wide range of genes were detected and used as diagnostic indices (see Japanese Unexamined Patent Publication No. 2003-245097). Yet, this report is focused on detection of variant genes upon sunlight exposure of skin. Thus, there has been a demand for a method of judgment or countermeasure based on an extensive analysis of skin spots per se.

Accordingly, the present inventors collected solar pigmented macules, typical skin spots, from 16 volunteers and compared them with adjacent sites using gene profile analysis (see Japanese Unexamined Patent Publication No. 2007-289063). The results have revealed that the skin spot sites involve, in addition to activation of melanin synthetic genes, activation of inflammation-related genes, repression of keratinization-related genes, and decrease in proliferation of keratinocytes.

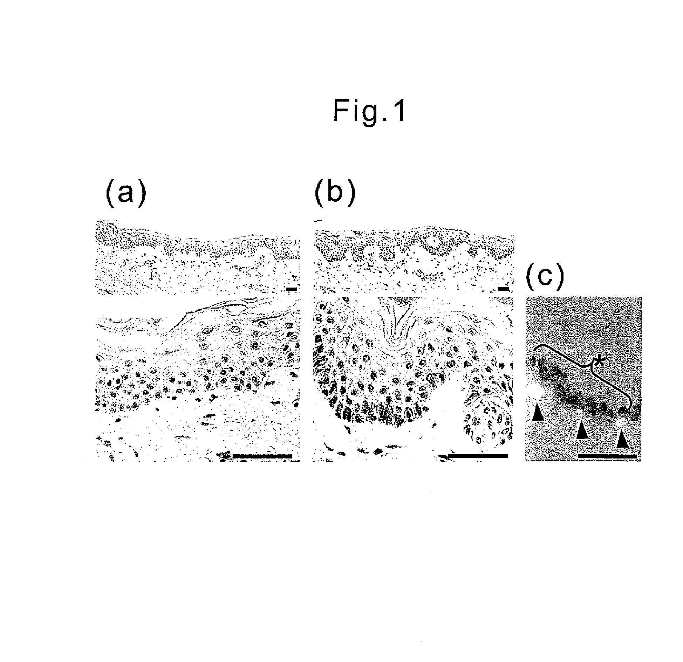

The amount of melanin in skin spot sites is significant larger than that in healthy sites. However, it has been reported that the number of melanocytes producing melanin in skin spot sites is merely about 1 to 2 times as many as that in healthy sites (see Cario-Andre M, Lepreux S, Pain C et al. Perilesional vs. lesional skin changes in senile lentigo. J Cutan Pathol., 31:441-7 (2004); Noblesse E, Nizard C, Cario-Andre M et al. Skin ultrastructure in senile lentigo. Skin Pharmacol Physiol, 19:95-100 (2006); Unver N, Freyschmidt-Paul P, Horster S et al. Alterations in the epidermal-dermal melanin axis and factor XIIIa melanophages in senile lentigo and ageing skin. Br J Dermatol., 155:119-28 (2006)). Also, histological observation of spot sites shows that melanocytes are rather transparent, while keratinocytes in the surrounding basal layer contain a large amount of melanin (see Figures 1 (b) and (c) described later). This indicates that coloring of skin spot sites is mainly due to black, melanin-containing keratinocytes which continuously receive melanin from melanocytes. A major question has been why these black keratinocytes keep staying in the epidermis of skin spot sites despite continuous regenesis of epidermis by turnover.

A possible cause of this long-term accumulation of melanin-containing keratinocytes is a decrease in turnover of the epidermis in skin spot sites. As a matter of fact, some papers have thus far reported the observation that the division of keratinocytes is reduced in skin spot sites (see Japanese Unexamined Patent Publication No. 2004-205246, Japanese Unexamined Patent Publication No. 2007-289063, Noblesse E, Nizard C, Cario-Andre M et al. Skin ultrastructure in senile lentigo. Skin Pharmacol Physiol, 19:95-100 (2006), Unver N, Freyschmidt-Paul P, Horster S et al. Alterations in the epidermal-dermal melanin axis and factor XIIIa melanophages in senile lentigo and ageing skin. Br J Dermatol., 155:119-28 (2006)). Yet, the details thereof, such as its relationship with melanin, are unknown.

Another known method for eliminating skin spots is enhancement of turnover (see Japanese Unexamined Patent Publication No. 6-72842, Japanese Patent Application Laid-Open Publication No. 6-145038, Japanese Patent Application Laid-Open Publication No. 2006-45084, Japanese Unexamined Patent Publication No. 2004-51544 and Japanese Unexamined Patent Publication No. 2001-302454). However, there is concern that simple enhancement of turnover might induce side effects such as skin damage or, if worst, inflammatory pigmentation. Thus, no applicable methods have not been found that can effectively eliminate skin spots.

There are also cosmetic medicine treatments for skin spot removal, such as chemical pealing, retinoic acid therapy or laser treatment (see Japanese Unexamined Patent Publication No. 2003-277251). These are medical practices of removing melanin-containing cells by promoting proliferation thereof or destroying them with exothermic heat by light absorption. Although promising a certain level of effect, they may also produce severe side effects such as pain, redness or swelling. Besides, they also involve high-frequency relapse occurred by post-inflammation pigmentation, as well as problems accompanied by hospital visit and medical cost.

[Disclosure of the Invention]

[Problems to Be Solved by the Invention]

In view of the above-mentioned problems, an object of the present invention is to provide a method of skin whitening which enables efficient prevention of skin spot formation and/or improvement of skin spots with reduced side effects, as well as to provide a screening method which can select, with high accuracy, a skin-spot-formation inhibiting and/or skin-spot removing factors with fewer side effects.

[Means for Solving the Problems]

In order to address these problems involves in conventional methods of skin whitening and to solve the questions concerning pigmentation phenomena, the present inventors carried out a thorough investigation to determine what differences are between skin spot sites and normal sites adjacent thereof, through gene profile analysis and immune antibody staining analysis of solar pigmented macules collected from 16 volunteers. As a result, the present inventors found that decrease in proliferation and/or differentiation (hereinafter also called "proliferation/differentiation") was not observed in all keratinocytes of the basal layer, but that only melanin-containing keratinocytes showed a significant decrease in proliferation/differentiation thereof.

Further, the present inventors simulated the conditions of the basal layer of skin spots by making cultured keratinocytes phagocytize melanin, and investigated the proliferation/differentiation properties thereof. As a result, the present inventors observed a similar decrease in proliferation/differentiation, as well as a decrease in, e.g., cyclins. These results suggest that in skin spot sites, proliferation/differentiation of melanin-containing keratinocytes in the basal layer is reduced, and that keratinocytes were not discharged by normal turnover and accumulated all over the basal layer, probably causing the chronic pigmentation in skin spot sites.

If turnover of epidermis is simply increased in order to get rid of skin spots, it may overstimulate the tendency of excessive proliferation/differentiation of normal keratinocytes (which tendency is essential in maintaining skin thickness), and may thereby induce side effects such as skin damage or, if worst, inflammatory pigmentation. On the other hand, it would be possible to effectively remove skin spots with suppressing the side effects by specifically enhancing only the turnover of black keratinocytes with sluggish proliferation/differentiation, which are causes of the pigmentation in skin spot sites and the chronicity thereof, to thereby discharge accumulated melanin and normalize the skin.

Based on the above findings, the present inventors have completed the present invention.

According to a first aspect, the present invention provides a method of skin whitening including the step of selectively activating proliferation and/or differentiation of melanin-containing keratinocytes of the skin.

According to a preferred embodiment, the above-mentioned step of selective activation is carried out by administrating a drug containing a skin-spot-formation inhibiting and/or skin-spot removing factor to a subject.

Further, according to a more preferred embodiment, the above-mentioned skin-spot-formation inhibiting and/or skin-spot removing factor is selected from the group consisting of *Artemisia* extract, *Artemisia capillaris* extract and Peach leaf extract.

According to a second aspect, the present invention provides a method for screening a skin-spot-formation inhibiting and/or skin-spot removing factor, including the steps of: making a population of cultured keratinocytes phagocytize microparticles to prepare a population of keratinocytes whose proliferation and/or differentiation are/is reduced; and evaluating a candidate compound for its ability to selectively activate proliferation and/or differentiation of this population to thereby select a compound having the ability of activation as the skin-spot-formation inhibiting and/or skin-spot removing factor.

According to a preferred embodiment, the above-mentioned microparticles are naturally-occurring or synthetic melanin, melanosomes or microbeads with uniform properties.

According to a preferred embodiment, the ability to activate proliferation is measured by a method selected from the group consisting of cell counting, alamar Blue assay, MTT (3-(4,5-dimethylthiazole-2-yl)-2,5-diphenyl tetrazolium bromide) assay, Hoechst assay, BrdU (5-bromo-2'-deoxyuridine) uptake measurement and immunohistochemical measurement using an antibody involved in cell proliferation, cell division or cell cycle.

According to a preferred embodiment, the ability to activate differentiation is measured by a method selected from cell morphology observation and immunohistochemical measurement using an antibody involved in cell differentiation.

According to a preferred embodiment, this method further comprises the steps of: applying a compound having the ability of activation to a skin model formed of melanin-containing keratinocytes to thereby select a compound having a skin-spot-formation inhibiting and/or skin-spot removing effect.

According to a preferred embodiment, this method further includes the steps of: constructing a three-dimensional skin model from the population of keratinocytes whose proliferation and/or differentiation are/is decreased; and evaluating the candidate compound using this three-dimensional skin model to thereby select a compound having a skin-spot-formation inhibiting and/or skin-spot removing effect.

[Effects of the Invention]

The method of skin whitening according to the present invention makes it possible to prevent formation of skin spots and/or improving existing skin spots in the skin efficiently while suppressing side effects.
The method for screening according to the present invention makes it possible to select, with high accuracy, a skin-spot-formation inhibiting and/or skin-spot removing factor with reduced side effects.

[Brief Description of the Drawings]

[Fig. 1] Figs. 1 (a) to 1 (c) are all micrographs of skin tissues. Fig. 1 (a) is an example of an HE-stained image of the skin tissue of a healthy site. Fig. 1 (b) is an example of an HE-stained image of the skin tissue of a solar pigmented macule site. Fig. 1 (c) is an example of a transmitted light image of the skin tissue of a solar pigmented macule site overlaid with an immunohistochemically-stained image thereof using a melanocyte marker (TRP-1).

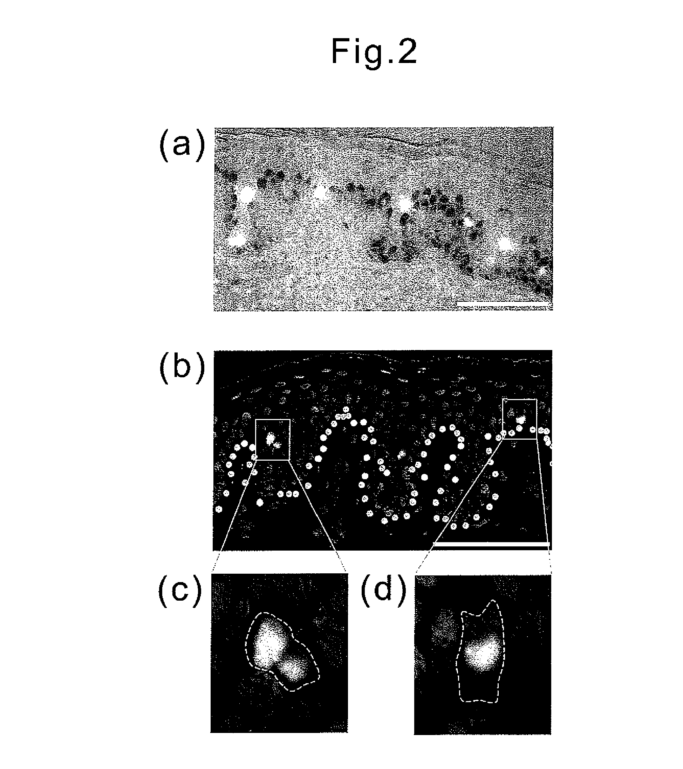

[Fig. 2] Figs. 2 (a) to 2 (c) are all stained images of dividing cells in tissues of spot sites of the skin. Fig. 2 (a) is an example of an immunohistochemically-stained image of a skin spot site tissue, in which the nuclei of dividing cells were stained with Ki67 staining (green). Fig. 2 (b) is an example of a triply-stained image of the spot site tissue, in which cell nuclei were stained with DAPI (blue), the nuclei of dividing cells were stained with Ki67 (green), and melanin was pusedo-color stained (red). Figs. 2 (c) and 2 (d) are figures partially magnifying an example of basal layer keratinocytes not containing melanin in Fig. 2 (b) and an example of basal layer keratinocytes containing melanin, respectively.

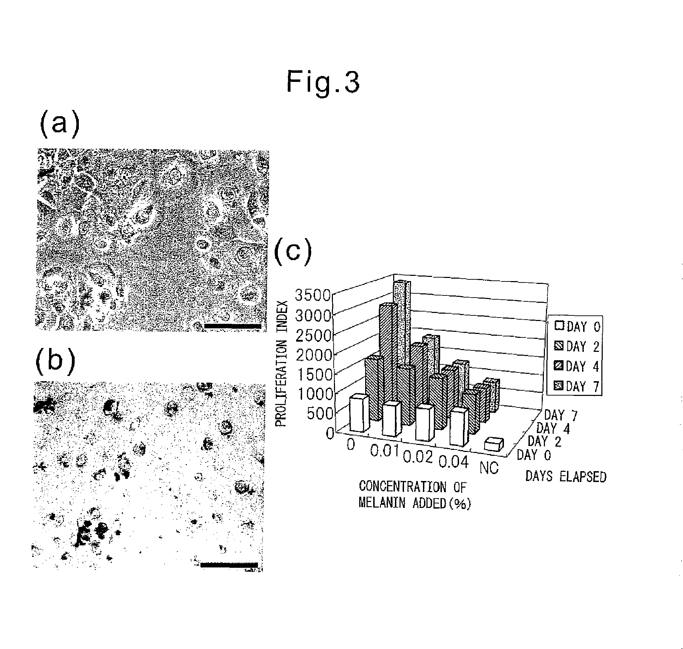

[Fig. 3] Figs. 3 (a) to 3 (c) are all figures to explain a decrease in proliferation of melanin-containing keratinocytes. Fig. 3 (a) is an example of a phase contrast micrograph of keratinocytes 3 days after addition of melanin source. Fig. 3 (b) is a transmitted light micrograph of the same visual field as Fig. 3 (a), melanin is shown in black. Fig. 3 (c) is a graph showing proliferation index (alamar Blue measurement value) at each melanin concentration 0, 2, 4, and 7 days after the addition of the melanin source.

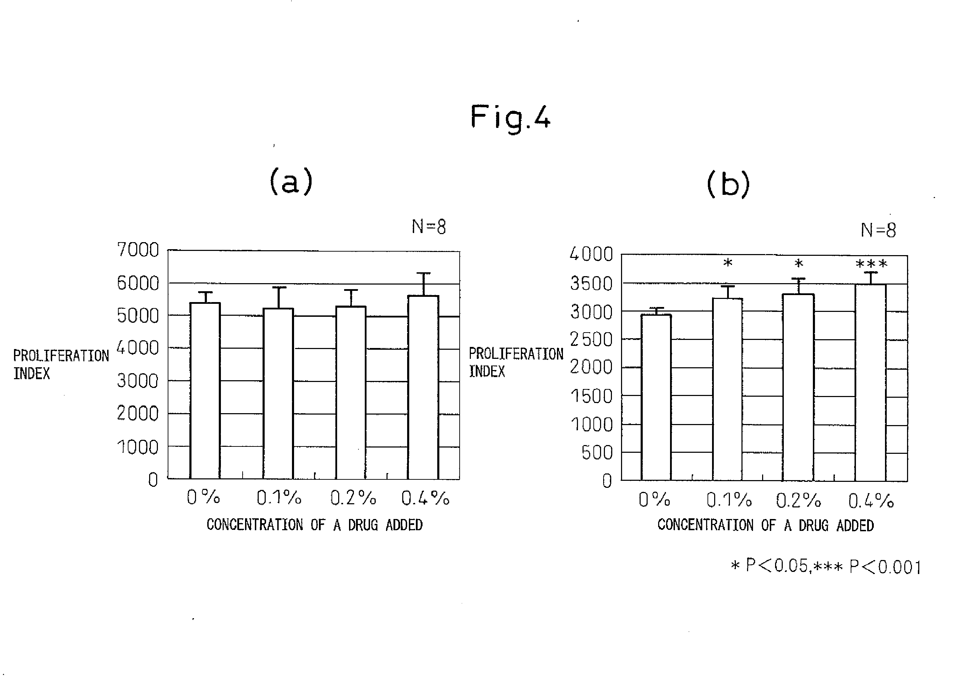

[Fig. 4] Fig. 4 (a) and Fig. 4 (b) are all figures showing influence of addition of *Artemisia* extract on melanin-containing keratinocytes. Specifically, Fig. 4(a) is a graph showing the values of the proliferation index (alamar Blue assay) when *Artemisia* extract was added to keratinocytes not-containing melanin. Fig. 4(b) is a graph showing the values of the proliferation index when *Artemisia* extract was added to melanin-containing keratinocytes.

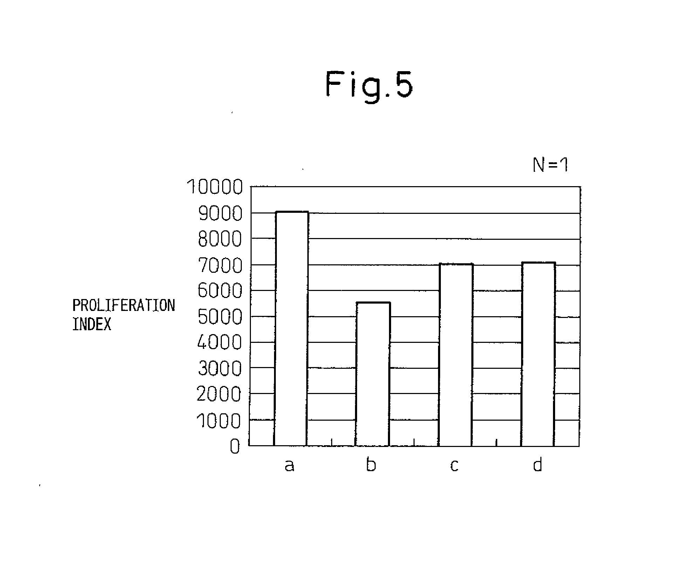

[Fig. 5] Fig. 5 is a graph of the proliferation index (alamar Blue assay) showing influence of *Artemisia capillaris* extract and peach leaf extract to microparticle (microbead)-containing keratinocytes. In this figure, bar (a) shows the result of keratinocytes not containing microbeads and bars (b) to (d) show the results (b) when no drugs were added, (c) when 0.2% *Artemisia capillaris* extract was added and (d) when 0.2% peach leaf extract was added to micorbeads-containing keratinocytes.

[Best Mode for Carrying out the Invention]

The present invention will be described in detail below by way of concrete embodiments thereof. Yet, the present invention is not limited thereto and can be worked with various modifications as long as it does not depart from the scope thereof.

1. Method of Skin Whitening:

The method of skin whitening according to the present invention includes the step of selectively activating proliferation/differentiation of keratinocytes containing melanin in the skin (hereinafter simply referred to as "melanin-containing keratinocytes"). In the present description, the term "method of skin whitening" refers to a method for preventing and/or removing excessive pigmentation in the skin by preventing spot formation in the skin and/or improving existing skin spots.
According to the above findings of the present inventors, among basal layer keratinocytes of the skin, proliferation/differentiation of melanin-containing keratinocytes specifically decreases, and melanin-containing keratinocytes stay all over the basal layer without being discharged by normal turnover, presumably causing chronic pigmentation of skin spot sites. However, if the turnover of various cells in skin spot sites is simply enhanced in a non-selective manner, excessive proliferation/differentiation of melanocytes would be overstimulated, so that skin spots would not be suppressed, or rather may be expanded. Also, if the turnover of basal layer keratinocytes is enhanced all together, it may overstimulate excessive proliferation/differentiation of normal keratinocytes (which is essential in maintaining the skin thickness), thereby inducing side effects such as skin damage and inflammatory pigmentation.
Contrary to this, the method of skin whitening according to the present invention normalizes melanin-containing keratinocytes by selectively activating the proliferation/differentiation of melanin-containing keratinocytes with sluggish proliferation/differentiation, which causes pigmentation of skin spot sites and chronicity thereof, and by specifically increasing the turnover of melanin-containing keratinocytes without increasing the turnover of, e.g., melanocytes and normal keratinocytes. This makes it possible to discharge melanin accumulated in skin spot sites, and to prevent spot formation in the skin while suppressing side effects and/or improving existing skin spots.

A specific method to selectively activate proliferation/differentiation of melanin-containing keratinocytes in the basal layer of the skin is exemplified by, although not limited to, the method which includes administrating a drug containing a compound having an ability to selectively activate the proliferation/differentiation of melanin-containing keratinocytes (skin-spot-formation inhibiting and/or skin-spot removing factor, which will be described later) to a subject. This skin-spot-formation inhibiting and/or skin-spot removing factor can be efficiently selected by the screening method according to the present invention. Examples of the skin-spot-formation inhibiting and/or skin-spot removing factor and the details of the screening method according to the present invention will be described in the next section "2. Method for Screening Skin-Spot-Formation Inhibiting and/or Skin-Spot Removing Factor." Also, a drug containing the skin-spot-formation inhibiting and/or skin-spot removing factor (composition for whitening, which will be described later) and a method of administration thereof will be described in the section after the next, "3. Compositions for Whitening".

2. Method for Skin-Spot-Formation Inhibiting and/or Skin-Spot Removing Factor:

The method for screening the skin-spot-formation inhibiting and/or skin-spot removing factor according to the present invention (hereinafter referred to as "the method of screening according to the present invention") includes the steps of: preparing a population of keratinocytes whose proliferation/differentiation is reduced, by making cultured keratinocytes phagocytize microparticles; and evaluating a candidate compound based on its ability to selectively activate the proliferation/differentiation of this population as an index to thereby select a compound having the ability of activation as the skin-spot-formation inhibiting and/or skin-spot removing factor.

In the present description, a factor having an action to suppress formation of spots in the skin and/or an action to remove existing spots in the skin is referred to as, e.g., an "inhibitory and/or removing factor for skin spot formation."

Microparticles are usually selected from naturally-occurring or synthetic melanin, melanosomes or microbeads with uniform properties.
Examples of melanin or melanosomes include: purified form of melanin and melanosomes extracted from cultured melanosites of, e.g., human, mouse, and cuttlefish. An example of a commercially available melanin is Sepia melanin (derived from cuttlefish) manufactured by Sigma.

The term "microbeads with uniform properties" refers to synthetic beads whose physiological properties (e.g., mass, particle diameter or degree of hardness) and chemical properties (e.g., surface reactivity or aggregability) have been equalized. Materials for the microbeads with uniform properties are exemplified by, although not limited to, one or more materials selected from plastics such as polystyrene or polyethylene.

The shape and size of the microbead are exemplified by, although not limited to, a spherical or substantially spherical shape having an average diameter of usually not less than 0.1 $\mu$m, preferably not less than 1 $\mu$m and usually not more than 10 $\mu$m, preferably not more than 3 $\mu$m. An example of commercially available microbeads with uniform properties is Polybeads from Polyscience.

The form of the microparticles should usually be, although not limited to, sufficiently small particles (e.g., with a diameter of usually not more than 2 $\mu$m), compared with the size of cells.

The percentage of the microparticles to be used should usually be, although not limited to, within such a range that apparent decrease in proliferation/differentiation is observed in the cells and that no significant cytotoxicity (e.g., peeling off of cells and lack of respiratory activity) occurs. Specifically, in cases where melanin is used as the microparticles, it is preferred that the percentage of melanin based on cell culture medium be usually not less than 0.0001% by mass, in particular not less than 0.001% by mass and usually not more than 0.1% by mass, in particular not more than 0.04% by mass.

Examples of a method for measuring the ability to activate proliferation include cell counting, alamar Blue assay, MTT assay, Hoechst assay, BrdU uptake measurement and immunohistochemical measurement using an antibody involved in cell proliferation, cell division or cell cycle (for example BrdU, Ki67, PCNA or the like). Conditions and procedures of these methods are well known to those skilled in the art.

Examples of a method for measuring the ability to activate differentiation include cell morphology observation and immunohistochemical measurement using an antibody involved in cell differentiation (e.g., keratin 1, keratin 10, filaggrin, involucrin, loricrin, and transglutaminase). Conditions and procedures of these methods are also well known to those skilled in the art.

In addition, it is preferred that the screening method according to the present invention further includes the step of applying the compound having the ability of activation to the skin model to thereby select a compound having a skin-spot-formation inhibiting and/or skin-spot removing effect. Examples of a skin model include: a monolayer culture, co-culture, or three-dimensional culture of skin cells containing melanin-containing keratinocytes; and a spot model mouse (see Japanese Patent Application Laid-Open Publication No. 2005-106745). Preferred among them is the monolayer culture of skin cells. Methods for constructing and using these skin models are also well known to those skilled in the art.

In particular, it is preferred that the screening method according to the present invention further includes the steps of: constructing a three-dimensional skin model using the above-mentioned population of keratinocytes whose proliferation/differentiation is reduced; and evaluating the above-mentioned candidate compound using this three-dimensional skin model to thereby select a compound having a skin-spot-formation inhibiting and/or skin-spot removing effect. Among the models described above, as three dimensional skin model, three dimensional culture, spot model mouse or the like is exemplified with the three dimensional culture being preferred.

The screening method of according to the present invention can specifically carried out, e.g., by the following steps.

(1) Human normal keratinocytes are cultured under appropriate conditions (e.g., under an atmosphere of 5% $CO_2$ in air at a temperature of 37°C and a humidity of 95% in an incubator for several days) using an appropriate culture medium (e.g., Defined Keratinocyte-SFM culture medium manufactured by Invitrogen, Gibco).

(2) A suspension of an appropriate concentration is prepared by dispersing microparticles in an appropriate buffer (e.g., PBS buffer) under sterilization.

(3) The medium of the cell culture from (1) is replaced with a culture medium containing the suspension from (2) at an appropriate concentration (e.g., a final concentration of melanin of 0.001 to 0.04% W/V based on the culture medium), and incubated (e.g., under the above-mentioned culture conditions for 2 to 3 days) to make keratinocytes to phagocytize melanin. Some cells are left as a control without being combined with microparticles.

(4) A monolayer culture product of keratinocytes whose proliferation/differentiation thereof is decreased by the operation of (3) is, as required, subjected to construction of a skin model. Thereafter, a culture medium of this monolayer culture product or skin model is replaced with a culture medium containing a test ingredient (candidate compound), and the culturing is further continued (e.g., in the case of the monolayer culture, under the above-mentioned culture conditions for 2 to 3 days). Some cells may be left as a control without being combined with test ingredients.

(5) The proliferation rate of the cells after the operation of (4) is measured.

(6) An ingredient (compound) which promotes proliferation of the keratinocytes with reduced proliferation/differentiation due to phagocytisis of microparticles is selected based on the measurement of (5). Further, taking into consideration effects of this ingredient on the proliferation rate of normal keratinocytes to which no microparticles were added, an ingredient having lower or no effects to promote the proliferation of normal cells is also selected.

(7) The differentiation index of the cells after the operation of (4) is measured.

(8) An ingredient which promotes differentiation of the keratinocytes with reduced proliferation/differentiation due to phagocysis of microparticles is selected based on the measurement of (7). At that time, taking into consideration effects of this ingredient on the increasing rate of differentiation index of normal keratinocytes to which no microparticles were added, an ingredient having lower or no effects to promote the differentiation of normal cells is selected.

As criteria for judging the promotion of proliferation and differentiation of keratinocytes, usually using a case where a test ingredient is not added as a standard, when a proliferation rate or differentiation rate statistically significantly increases by addition of the test ingredient, the proliferation or differentiation is judged to be promoted by this ingredient. It should be noted that the addition of test ingredient is carried out within a concentration range where no significant cytotoxicity occurs.

The screening method of the present invention described above makes it possible to select, with high accuracy, a skin-spot-formation inhibiting and/or skin-spot removing factor with fewer side effects, by evaluating a candidate compound using, as an index, the ability to selectively activate proliferation/differentiation of a population of keratinocytes whose proliferation/differentiation is reduced. Further, combining evaluations using two or more skin models enables a compound having a skin-spot-formation inhibiting and/or skin-spot removing effect with fewer side effects to be selected with high accuracy.

It is expected that an ingredient (compound) selected as a skin-spot-formation inhibiting and/or skin-spot removing factor according to the screening method of the present invention can effectively prevent or improve skin spots with fewer side effects by specifically restoring the proliferation/differentiation of black keratinocytes (whose proliferation/differentiation is reduced due to accumulation of melanin and cause chronic pigmentation of skin spot sites) to the normal state and by releasing the accumulated melanin. In addition, it is expected to enables removal of existing skin spots (i.e., the black keratinocytes), which are difficult to remove with, e.g., a tyrosinase inhibitor. Therefore, the skin-spot-formation inhibiting and/or skin-spot removing factor selected by the screening method of the present invention is expected to be used as an effective ingredient for whitening.

The present inventors carried out a selection for a skin-spot-formation inhibiting and/or skin-spot removing factor using the screening method of the present invention described above and, as a result, found that *Artemisia* extract, *Artemisia capillaris* extract and peach leaf extract have effects to promote proliferation/differentiation of melanin-containing keratinocytes.

Both wormwood (scientific name: *Artemisia princeps*) and capillary wormwood (scientific name: *Artemisia capillaris*) are perennials belonging to the genus *Artemisia* within the family *Asteraceae.*

A "peach leaf" refers to a leaf of peach (scientific name *Amygdalus persica*) which is a deciduous tree with a height of 5 to 10 m belonging to the genus *Amygdalus* within the family *Rosaceae.*

The "extract" of wormwood, capillary wormwood and peach leaf can be obtained by a conventional method, e.g., by immersing these plants in, or heating them under reflux with, an extraction solvent, and filtrating and concentrating the resultant. An extraction solvent may be any solvent as long as it can usually be used for extraction. Examples thereof include: water; alcohols such as methanol, ethanol, propylene glycol, 1,3-butylene glycol or glycerin; aqueous alcohol; organic solvents such as chloroform, dichloroethane, carbon tetrachloride, acetone, ethyl acetate or hexane. These solvents can be used either singly or in combination of any two or more thereof. An extract with the above-mentioned solvent can be used either without modification or after undergoing post-treatment. Examples of post-treatment include: concentration thereof; removal of insoluble matters therefrom by adsorption with, e.g., ion-exchange resins; and adsorption by a column of a porous polymer (for example, Amberlites XAD-2); extraction using methanol or ethanol; and a partition using, e.g., water/ethyl acetate.

3. Compositions for Whitening:

A skin-spot-formation inhibiting and/or skin-spot removing factor selected by the screening method of the present invention (e.g., the above-described *Artemisia* extract, *Artemisia capillaris* extract, peach leaf extract) can usually be used, as an effective whitening ingredient in a composition for whitening (or external preparation for skin whitening), in the method of skin whitening of the present invention described above. The composition for whitening which contains the skin-spot-formation inhibiting and/or skin-spot removing factor selected by the screening method of the present invention as an effective whitening ingredient is referred to herein as a "composition for whitening of the present invention."

The percentage of the skin-spot-formation inhibiting and/or removing factor to the composition for whitening of the present invention is not particularly limited since it may vary depending on the formulation or dosage thereof. Preferably, the percentage should be in a range between usually not less than 0.001% by mass, in particular not less than 0.01% by mass, further not less than 0.1% by mass and usually not more than 10% by mass, in particular not more than 5% by mass, further not more than 1% by mass.

The composition for whitening of the present invention, depending on a desired formulation, may contain an additional ingredient such as conventionally known vehicles, flavors or the like as well as oils and fats, detergents, antiseptics, sequestering agent, water soluble polymers, thickeners, powdered ingredients such as pigments, ultraviolet-ray protective agents, moisturizers, antioxidants, pH adjusters, cleaning agents, desiccants, emulsifiers. Further, as long as it does not adversely affect an intended effect, other pharmacologically active ingredients can be also added to the composition for whitening of the present invention.

The composition for whitening of the present invention are applied, for example, in the form of aqueous solution, oily solution, other solutions, emulsion, cream, gel, suspension, microcapsule, powder, granule, capsule, solid preparations. After prepared into these forms by a conventionally-known method such as lotion preparations, emulsion agents, creams, ointments, plasters, cataplasms, aerosols, water-oil two layer systems, water-oil-powder three layer systems, injection solutions, oral drugs (e.g., tablets, powders, pellets, balls, syrups, and troches), and suppositories, the composition can be administrated to the body of a subject such as human or an animal by, e.g., application, attaching, spraying, injecting, drinking, or insertion.

Among these forms, preparing the composition for whitening of the present invention in the form of an external preparation for skin, such as lotion preparation, emulsion, cream, ointment, plaster, cataplasm or aerosol is suitable for the object of the present invention. The composition for whitening of the present invention prepared in the form of skin external preparation is referred to herein as an "external preparation for skin whitening of the present invention".

The external preparations for skin described herein include drugs, quasi drugs (e.g., ointments), cosmetics [basic cosmetics such as facial wash, emulsion, cream, gell, essence (beauty essence) or pack mask; makeup cosmetics such as foundation or lip stick; oral cavity cosmetics; aroma cosmetics; hair cosmetics; and body cosmetics]. In particular, the composition for whitening of the present invention should preferably be applied as a cosmetic to prevent skin spot formation and/or improve skin spots.

By administrating the composition for whitening of the present invention (in particular, the external preparation for skin whitening of the present invention) to a subject in an appropriate method depending on the formulation, it is possible to effectively prevent skin spot formation and/or improve skin spots while suppressing side effects.

[EXAMPLES]

The present invention will be now described in more detail by way of examples, although the present invention is not limited to these examples.

[Example 1]

Results of Analysis of Human Skin Spot Tissues

Solar Pigmented Macule Site Tissue Sample

A solar pigmented macule was selected, based on judgment by a dermatologist, from a skin spot present in the back of each of 16 male volunteers in their 40s or older who were given informed consent. Epidermis and upper dermis tissues of the solar pigmented macule site were collected by 3 mm biopsy under local anesthesia to provide a tissue sample. The collection was carried out in accordance with the guideline by Shiseido ethical committee. Besides from the solar pigmented macule site, epidermis and upper dermis tissues were also collected in a similar manner from, as comparison sites, an adjacent healthy site in the back and a healthy site in the hip (site which was not exposed to light) of the identical subject.

Staining of Skin Tissue Section

In order to observe distribution and expression of changed proteins inside the skin tissue of the skin spot site as compared with those from the comparison sites, the collected tissues were made into frozen blocks to prepare thin slice sections, which were subjected to various cell staining operations shown below, such as HE staining or immunohistochemical staining. To avoid fluctuations, tissues of the three sites (the solar pigmented macule site, the healthy site, and the site which was not exposed to light) from an identical subject were embedded together in one block, which was placed on one slide and simultaneously subjected to a staining operation.

Immunohistochemical staining was carried out together with Tyramide Signal Amplification (TSA-plus Fluorescein System: Perkin Elmer Life and Analytical Sciences, Boston, MA, U.S.A.), using the anti-TRP-1 antibody (see Me-5: Signet Laboratories Inc., Dedham, MA, U.S.A.) and the Ki-67 antibody (BD Biosciences Pharmingen, San Diego, CA, U.S.A.). The number of positive cells was counted per millimeter of length of the epidermis.

Results of Analysis

HE (Hematoxylin-Eosin) staining was first carried out for each site. Fig. 1 (a) is an example of an HE-stained image of a skin tissue in a healthy site. Fig. 1(b) is an example of an HE-stained image of a skin tissue in a solar pigmented macule site. Each of Figs 1 (a) and (b) shows vertically-overlaid two images with different magnifications, and the length of a black line in each image is equivalent to 50 $\mu$m.

Subsequently, in order to check an increase of a melanocyte-related gene in the spot site, an expression pattern of the TRP-1 protein, which is a melanocyte marker, in the tissue was examined by immunohistochemical staining using the anti-TRP-1 antibody. Cells emitting TRP-1-positive fluorescence signals were detected in melanocytes in the basal membrane. In 9 out of 9 examples, it was confirmed that the number of the cells emitting the fluorescence signals per unit length of the stratum corneum in the solar pigmented macule site is about twice as many as that in the adjacent healthy site. This agrees to the published results described in the above "Background Art" section.

Fig. 1 (c) is an example of the thus-obtained immunohistochemically stained image of the skin tissue of a solar pigmented macule site by a melanocyte marker (TRP-1) overlaid with a transmitted light image of the skin tissue in the identical site. In Fig. 1 (c), the length of the black line is equal to 50 $\mu$m. Arrows indicate TRP-1 positive melanocytes (green) and asterisks (*) indicate basal layer keratinocytes containing melanin (black). Fig. 1 (c) suggests that the melanocytes were rather transparent, while the basal layer keratinocytes exhibited black and contained melanin.

Next, cells under proliferation were detected using the Ki67 antibody, which is a cell division maker, and a similar comparison was carried out. As a result, contrary to the above, it was observed that the number of positive cells in the solar pigmented macule site tend to decrease, being 0.58 times (n=10, p=0.021) as many as that in the adjacent healthy site. Most of these positive cells are keratinocytes. Fig. 2 (a) is an example of an immunohistochemical staining (Ki67 staining) image of the spot site tissue, in which image the nuclei of dividing cells were stained green. In Fig. 2 (a), the length of the white line is

equal to 100 μm.

The reason why the spot site contained less positive cells was assumed to be due to a light shading effect of melanin on fluorescence signals. Therefore, this image of the Ki67 positive cells was closely observed. The observation revealed that although the signals of Ki67 were detected in the cell nuclei, melanin was distributed in the perinuclear cytoplasm, showing that shading of the fluorescence signals did not occur so much as anticipated. Rather, close observation of the perinuclear regions of the nuclei in the positive cells, where melanin supposed to be distributed, revealed that most of the Ki67 positive cells (i.e. dividing cells) contained almost no melanin therein (regions surrounding the nuclei, which were stained green), as shown in Fig. 2(a).

Subsequently, in order to investigate whether or not these phenomena were specific to skin spot sites, the following measurement was carried out.

First, in order to judge the localization of cells in the tissue sections from the skin spot site, a triply-stained image was prepared by staining the nuclei blue with DAPI (4',6-diamidino-2-phenylindole), staining dividing cells green with Ki67, and staining melanin red with a pseudo color, thereby enabling the discrimination between these three. In the thus-obtained triply-stained image of the spot site tissue, the total number of basal layer cells containing melanin and the total number of basal layer cells not containing melanin were counted per unit length of the stratum corneum. In addition, the number of Ki67-positive dividing cells among each of these cell groups was counted, and by dividing it either by the total numbers of the basal layer cells containing melanin or by the total number of basal layer cells not containing melanin, a percentage of the dividing cells in each cell group was calculated.

Fig. 2 (b) is an example of the triply-stained image of the spot site tissue. The basal layer cells not containing melanin and the basal layer cells containing melanin are indicated with white circles and pink circles, respectively. In addition, magnified part views of an area boxed with the white lines in Fig. 2 (b) (an example of the basal layer keratinocytes not containing melanin and an example of the basal layer keratinocytes containing melanin) are shown in Figs. 2 (c) and (d), respectively. In Figs. 2 (c) and (d), the outlines of keratinocytes are shown with white dotted lines.

As for the criteria for discriminating cells not containing melanin from cells containing melanin, cells in which melanin was virtually not observed (e.g., cells shown in Fig. 2 (c)) were judged as the cells not containing melanin whereas other cells (e.g., cells shown in Fig. 2(d)) were judged as the cells containing melanin. Also, although Ki67 positive cells may in some cases be detected not in the basal layer but in a layer immediately above the basal layer due to the characteristics of these cells, the calculation was carried out assuming that the cells were detected in the basal layer.

The percentage of Ki67 positive cells either in basal layer cells containing melanin and basal layer cells not containing melanin was calculated in accordance with formulae (1) and (2), respectively.

$$Rm=Dm/Bm \quad (1)$$

In formula (1), Rm represents the percentage (Ratio) of Ki67 positive cells in basal layer cells containing melanin (melanin); Dm represents the number of Ki67 positive dividing cells among the basal layer cells containing melanin (Divide); and Bm represents the total number of basal layer cells containing melanin (Basal).

$$Rc=Dc/Bc \quad (2)$$

In formula (2), Rc represents the percentage of Ki67 positive cells in basal layer cells not containing melanin (clear); Dc represents the number of Ki67 positive dividing cells among the basal layer cells not containing melanin; and Bc represents the total number of basal layer cells not containing melanin.

In accordance with the above procedures, the number of basal layer cells containing melanin and the number of basal layer cells not containing melanin (in Fig. 2 (b), the number of the cells marked with white circles and the number of the cells marked with pink circles, respectively) was counted. The percentage (Rm, Rc) of dividing cells (in Fig. 2 (b), cells having the nuclei stained green) either among the melanin-containing cells and among the cells not containing melanin were calculated and compared. As a result, the percentage of dividing cells in the basal layer cells containing melanin was significantly lower than the percentage of dividing cells in the basal layer cells not containing melanin, suggesting that division of melanin-containing keratinocytes was significantly reduced (Rm/Rc=0.16 folds, N=14, P=0.007).

That is, it was thought that the basal layer keratinocytes storing melanin in the skin spot sites were in the state where proliferation/differentiation was significantly reduced. These black keratinocytes which hardly divide will continue to stay in the basal layer for a long period of time unlike normal keratinocytes, which undergo continuous replacement by division and turnover in the basal layer. This will prolong the physical time necessary to transfer melanin from melanocytes and will keep a high content of melanin. The presence of such black keratinocytes, whose proliferation/differentiation is reduced, all over the basal layer was assumed as a cause of the chronic pigmentation of skin spot sites. As a matter of fact, observation of the images of tissues stained by TRP-1 (see Figs. 1 (b) and 1 (c) described above) reveals that the melanin-containing keratinocytes are distributed all over the basal layer to be a main cause of the coloring of skin spot site.

[Example 2]

Results of Analysis Using Cultured Human Normal Keratinocytes

In order to check whether or not the decrease of proliferation/differentiation of melanin-containing keratinocytes observed in the analysis of skin spot site tissues (*in vivo*) was also observed in a cultured cell (in *vitro*) system, a condition similar to the basal layer of skin spot sites was prepared by allowing cultured human normal keratinocytes to phagocytize microparticles subjected to analysis.

Cell Culture and Addition of Microparticles

(1) Human normal keratinocytes were cultured under an atmosphere of 5% $CO_2$ in air at a temperature of 37°C and a humidity of 95% in an incubator using Defined Keratinocyte-SFM culture medium (manufactured by Invitrogen, Gibco).

(2) Using melanin (Sepia melanin (derived from cuttlefish), powder, manufactured by Sigma) as microparticles, a suspension of an appropriate concentration was prepared by dispersing melanin in a PBS buffer under sterilization.

(3) The medium of the cell culture of (1) was replaced with a culture medium containing the suspension of (2) (at a final concentration of melanin of 0.001, 0.02, and 0.04% W/V based on the culture medium) and incubated to allow keratinocytes to phagocytize melanin. After 1 to 3 days under the above-mentioned culture conditions, the medium with melanin was replaced with a fresh culture medium not containing melanin to wash off extra melanin. Culturing was continued further while the medium was periodically (every two to three days) replaced.
Meanwhile, cells to which no melanin was added were also prepared as a control.

Observation of Division and Measurement of Proliferation Rate

(1) After the addition of melanin, a phase contrast micrograph of cell conditions and transmitted-light micrograph with no phase contrast (to clearly look at melanin distribution) were periodically taken to observe the state of cell division.

(2) After the addition of melanin, a cell proliferation rate was periodically measured by alamar Blue assay. In the alamar Blue assay, a 1/10 volume of alamar Blue solution (Bio Source) was added to the cell culture medium and then left to stand in a culture incubator for one hour. Thereafter, part of the culture supernatant was collected and transferred to, e.g., a 96-well plate, and excitation was carried out at a wavelength of 544 nm, followed by measurement at a wavelength of 590 nm using a fluorescence plate reader. The cell culture medium (solution) after the addition of the alamar Blue solution and the measurement was immediately replaced with a fresh culture medium to continue the culturing.

Analysis of Changed Genes in Melanin-Added Cells.

(1) From human normal keratinocytes, to which melanin was added, in the state in which division thereof is decreased, as well as from the control to which melanin was not added, RNA was extracted by ISOGEN (Nippon Gene Co., Ltd., the protocol recommended by the manufacturer) and purified by RNeasy (Qiagen). The obtained RNA was subjected to gel electrophoresis using a bioanalyzer (Agilent Technologies) to check the quality and the degree of purification.

(2) Using the prepared RNA, gene profile analysis was carried out by DNA microarray(s). Preparation of the samples and hybridization were carried out in accordance with the protocol recommended by Agilent. From a total amount of 100 to 300 ng of RNA, labeled cRNA was synthesized using Low RNA Input Linear amplification and labeling kit (Agilent) and purified by RNeasy (Qiagen, the protocol recommended by the manufacturer). Thereafter, hybridization with Whole Human Genome oligo array (Agilent, G4112A) and wash were carried out and then data were read by a scanner of Agilent.

Analysis of Changed Protein in Melanin-Added Cells

From human normal keratinocytes, to which melanin was added, in the state in which division thereof is decreased, as well as from the control to which melanin was not added, proteins were extracted. The amount of target proteins was compared by Western blotting.

Results of Analysis

An example of a phase contrast micrograph of keratinocytes 3 days after the addition of melanin is shown in Fig. 3 (a), and a transmitted-light micrograph of the same visual field as Fig. 3 (a) is shown in Fig. 3 (b). In Fig. 3 (b), melanin is shown in black. In Figs. 3 (a) and (b), the length of the black line is equal to 100 $\mu$m. As shown in Figs. 3 (a) and (b), when melanin was added to human normal keratinocytes, melanin was quickly phagocytized and then accumulated in the perinuclear region. Thereafter, after the second day of the culturing, sluggish cell division was observed by the micrograph.

Fig. 3 (c) is a graph showing cell proliferation index (alamar Blue measured value) at each melanin concentration 0, 2, 4 and 7 days after the addition of melanin. As shown in Fig. 3 (c), in the case of no addition of melanin (at a melanin concentration of 0.00%) the proliferation of cells was observed to the 7th day of the culturing. In the case of a melanin concentration of 0.01%, a decrease in the cell proliferation was observed starting the 4th day. When the melanin concentration was increased to 0.04%, a significant decrease in the cell proliferation in a concentration-dependent fashion was observed starting the 2nd day after the addition. When the melanin concentration was 0.04%, alamar Blue values after the 2nd day were almost unchanged and the values virtually stayed at the value at the time of the addition of melanin. Since alamar Blue detects respiratory activities of the cells, it is understood that the cells did not become dead, i.e., that the cells were living in a static state without proliferating or differentiating.

In order to check if the addition of melanin did not exert influence such as adsorption of ingredient of the culture medium to melanin, the culture medium was in advance incubated with melanin (for 3 days) and cells were cultured using the culture medium in which melanin was removed. When observations were made, such influence did not appear in the proliferation of the cells.

In addition, using microbeads (polystyrene beads of an average diameter of 2 $\mu$m, purchased from Polyscience) and melanosomes (prepared from human normal melanocytes by ultracentrifugation fractionation) as microparticles, the same experiment as above was carried out and it was observed that the same phenomena again occurred.

In order to analyze in more detail the state of keratinocytes which phagocitized melanin and underwent decreased division, total RNA was extracted from both keratinocytes exhibiting decreased division and normal keratinocytes, and compared gene expression profiles thereof. For the analysis, a DNA microarray in which all human genes were listed was used. As a result, it was revealed that, among genes related to cyclins necessary for cell division, expression of many genes was reduced. PCNA which is a cell proliferation marker also decreased to 0.53 folds. Further, when a cyclin inhibitor group was looked at, it was clarified that expression of these cyclin inhibitors, contrary to that of the above genes, increased.

In order to investigate whether or not the decrease of these cyclins occurred at a protein level, protein expression of cyclin D and cyclin B was examined using Western blotting. As a result, it was confirmed that the expression of these cyclins was decreased at the protein level as well.

From the above results, it is suggested that the decrease in the division of the keratinocytes storing melanin is not simply toxicity but rather an active phenomenon of decreased division in which cell cycle determination mechanisms are involved.

Thus, the result that the division of cultured keratinocytes which were allowed to phagocytize melanin decreased finely agrees to the above-described observation findings that the division of the basal layer keratinocytes storing melanin in skin spot sites decreased. Therefore, it is considered that the phenomenon of decreased proliferation and division of the melanin-containing keratinocytes observed in skin spot tissues was also reproduced in a system of cultured cells. The fact that such black keratinocytes which store melanin and do not proliferate or differentiate, and whose proliferation and differentiation are decreased are present all over the basal layer is thought to be a main cause of the chronic pigmentation in skin spot sites.

[Example 3]

Screening of Drugs to Normalize Division of Melanin-Containing Keratinocytes

In the same procedures as Example 2, a condition where cell division was decreased was prepared by allowing cultured human normal keratinocytes to phagocytize microparticles; and using normalization thereof, that is, increase in cell division as an index, a system for drug screening was constructed and drug screening was attempted using the system.

Construction of Screening System

(1) Human normal keratinocytes were cultured under an atmosphere of 5% $CO_2$ in air at a temperature of 37°C and a humidity of 95% in an incubator using Defined Keratinocyte-SFM culture medium (manufactured by Invitrogen, Gibco). Additionally, human normal melanocytes were cultured under the same environment as above using Medium 254 + HMGS culture medium (KURABO).

(2) Using melanin (Sepia melanin manufactured by Sigma (derived from cuttlefish), powder) as microparticles, a suspension of an appropriate concentration was prepared by dispersing melanin in a PBS buffer under sterilization.

(3) The medium of the cell culture of (1) was replaced with a culture medium in which the suspension of (2) was added (a final concentration of melanin of 0.001 to 0.04% W/V based on the culture medium, a concentration at which cells proliferated to about half in number) and incubated to allow the cells to phagocytize melanin for 3 days (It was confirmed in advance by the same method as Example 2 that the addition of melanin did not exert influence such as adsorption of ingredients of the culture medium to melanin.). Thereafter, the medium with melanin was replaced with a fresh culture medium not containing melanin to wash off extra melanin. To this, drugs (candidate compounds) to be subjected to evaluation, which drugs will be described later, were added at varied concentration (0.1 to 0.8% W/V). The culturing was further continued under the above-mentioned culture conditions for 2 to 3 days. At that time, as a control, cells to which melanin was not added were prepared and treated in the same manner.

(4) 2 to 3 days after the addition of the drug, a cell proliferation index was measured by alamar Blue assay. In the alamar Blue assay, a 1/10 volume of alamar Blue solution (Bio Source) was added to the cell culture medium and then left to stand in a culture incubator for 1 hour. Thereafter, part of the culture supernatant was collected and transferred to a 96-well plate, and excitation was carried out at a wavelength of 544 nm, followed by measurement at a wavelength of 590 nm using a fluorescence plate reader.

Drug Screening

For about 200 drugs including various plant extracts, the screening was actually carried out using the above-mentioned screening system. As a result, the *Artemisia* extract, the *Artemisia capillaris* extract and the peach leaf extract were found to have the effect to promote the division of melanin-containing keratinocytes (These drugs were appropriately referred to as "selected drugs".).

Among the selected drugs, a graph of the proliferation index (alamar Blue assay) when the *Artemisia* extract was added to keratinocytes not containing melanin is shown in Fig. 4 (a), and a graph of the proliferation index when the extract was added to melanin-containing keratinocytes is shown in Fig. 4 (b). It is found that the *Artemisia* extract does not promote the proliferation thereof keratinocytes not containing melanin, whereas it has the effect to promote the cell proliferation for melanin-containing keratinocytes.

Fig. 5 is a graph of the proliferation index (alamar Blue assay) showing influence of other selected drugs namely *Artemisia capillaris* extract and peach leaf extract on keratinocytes containing microbeads as microparticles. In the graph, Bar (a) shows a value of the proliferation index (alamar Blue assay) in cases where keratinocytes not containing microbeads were cultured under the condition where no selected drugs were added. Bars (b), (c) and (d) show values of the proliferation index (alamar Blue assay) when (b) no selected drugs, (c) 0.2% *Artemisia capillaris* extract and (d) 0.2% peach leaf extract were added to microbeads-containing keratinocytes obtained by using microbeads ($\phi$ 2 $\mu$m, $2.2\times10^7$ beads/ml of culture medium) as microparticles. Fig. 8 shows that the *Artemisia capillaris* extract and the peach leaf extract have the effect to promote the proliferation for keratinocytes whose proliferation was reduced due to phogocysis of the microbeads.

From the above results, the selected drugs obtained by using the above-mentioned screening system specifically divide keratinocytes containing melanin and exhibiting decreased division. On the other hand, they do not exert such influence over normal keratinocytes and melanocytes. That is, the drugs can exsert the action to enhance proliferation specifically on melanin-containing keratinocytes, which are a main cause of the chronic pigmentation in skin spot sites. Therefore, it is considered that use of these selected drugs makes it possible to effectively improve skin spots while suppressing side effects.

[Industrial Applicability]

The present invention can be suitably used in the fields related to pharmaceuticals or cosmetics, particularly in the fields of pharmaceuticals or cosmetics to prevent skin spot formation and/or improve skin spots.

**Claims**

1. A method of skin whitening comprising:

   selectively activating proliferation and/or differentiation of melanin-containing keratinocytes of the skin.

2. A method for screening a skin-spot-formation inhibiting and/or skin-spot removing factor, comprising:

   introducing microparticles into a population of cultured keratinocytes to prepare a population of keratinocytes whose proliferation and/or differentiation are/is reduced; and evaluating a candidate compound based on its ability to selectively activate proliferation and/or differentiation of this population as an index to thereby select a compound having the ability of activation as the skin-spot-formation inhibiting and/or skin-spot removing factor.

3. The method according to claim 2, wherein the microparticles are naturally occurring or synthetic melanin, melanosomes, or microbeads with uniform properties.

4. The method according to claim 2, wherein the ability to activate proliferation is measured using a method selected from the group consisting of cell counting, alamar Blue assay, MTT (3-(4,5-dimethylthiazole-2-yl)-2,5-diphenyl tetrazolium bromide) assay, Hoechst assay, BrdU (5-bromo-2'-deoxyuridine) uptake measurement, and immunohistochemical measurement using an antibody involved in cell proliferation, cell division or cell cycle.

5. The method according to claim 2, wherein the ability to activate differentiation is measured by a method selected from the group consisting of cell morphology observation and immunohistochemical measurement using an antibody involved in cell differentiation.

6. The method according to claim 2, further comprising: applying a compound having the ability of activation to a skin model comprising melanin-containing keratinocytes to thereby select a compound having a skin-spot-formation inhibiting and/or skin-spot removing effect.

7. The method according to claim 2, further comprising: constructing a three-dimensional skin model from the population of keratinocytes whose proliferation and/or differentiation are/is reduced; and evaluating the candidate compound using this three-dimensional skin model to thereby select a compound having a skin-spot-formation inhibiting and/or skin-spot removing effect.

# Fig.1

(a)

(b)

(c)

# Fig.2

(a)

(b)

(c)

(d)

# Fig.3

(a)

(b)

(c)

# Fig.4

## (a)

N=8

PROLIFERATION INDEX

CONCENTRATION OF A DRUG ADDED

## (b)

N=8

PROLIFERATION INDEX

CONCENTRATION OF A DRUG ADDED

$* P < 0.05, *** P < 0.001$

EP 2 250 993 A1

# Fig.5

| INTERNATIONAL SEARCH REPORT | International application No. |
|---|---|
| | PCT/JP2009/054199 |

A. CLASSIFICATION OF SUBJECT MATTER
*A61K8/00*(2006.01)i, *A61K8/97*(2006.01)i, *A61Q19/02*(2006.01)i, *C12Q1/02*
(2006.01)i, *G01N33/15*(2006.01)i, *G01N33/50*(2006.01)i

According to International Patent Classification (IPC) or to both national classification and IPC

B. FIELDS SEARCHED

Minimum documentation searched (classification system followed by classification symbols)
A61K8/00, A61K8/97, A61Q19/02, C12Q1/02, G01N33/15, G01N33/50

Documentation searched other than minimum documentation to the extent that such documents are included in the fields searched
Jitsuyo Shinan Koho          1922-1996   Jitsuyo Shinan Toroku Koho   1996-2009
Kokai Jitsuyo Shinan Koho    1971-2009   Toroku Jitsuyo Shinan Koho   1994-2009

Electronic data base consulted during the international search (name of data base and, where practicable, search terms used)
BIOSIS(STN), CAplus(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 11-349435 A (Noevir Co., Ltd.),<br>21 December, 1999 (21.12.99),<br>Claims<br>(Family: none) | 1<br>2-7 |
| X<br>A | JP 8-104646 A (Noevir Co., Ltd.),<br>23 April, 1996 (23.04.96),<br>Claims<br>(Family: none) | 1<br>2-7 |
| X<br>A | JP 2003-342155 A (Noevir Co., Ltd.),<br>03 December, 2003 (03.12.03),<br>Claims<br>(Family: none) | 1<br>2-7 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 02 June, 2009 (02.06.09) | 16 June, 2009 (16.06.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2009/054199

C (Continuation).   DOCUMENTS CONSIDERED TO BE RELEVANT

| Category* | Citation of document, with indication, where appropriate, of the relevant passages | Relevant to claim No. |
|---|---|---|
| X<br>A | JP 2005-23042 A  (Noevir Co., Ltd.),<br>27 January, 2005 (27.01.05),<br>Claims<br>(Family: none) | 1<br>2-7 |
| X<br>A | JP 2006-176436 A  (Kao Corp.),<br>06 July, 2006 (06.07.06),<br>Claims<br>(Family: none) | 1<br>2-7 |
| A | WO 2007/126104 A1  (Shiseido Co., Ltd.),<br>08 November, 2007 (08.11.07),<br>Full text<br>& JP 2007-289063 A    & EP 2011883 A1 | 1-7 |

Form PCT/ISA/210 (continuation of second sheet) (April 2007)

| **INTERNATIONAL SEARCH REPORT** | International application No. |
| --- | --- |
| | PCT/JP2009/054199 |

<Subject of search>
    Claim 1 relates to "a skin whitening method comprising a step for selectively activating the proliferation and/or differentiation of melanin-containing keratinocytes in the skin".

    Although claim 1 involves any methods having an effect of "selectively activating the proliferation and/or differentiation of melanin-containing keratinocytes in the skin", only small part of the claimed methods, i.e., the functional effects of plant extracts prepared from mugwort (*Artemisia princeps*), *Artemisia capillaris Thumb* and peach leaves are merely disclosed in the meaning within PCT Article 5. Therefore, it appears that claim 1 is not supported by the disclosure of the description in the meaning within PCT Article 6.

    Although the common technical knowledge at the point of the application is taken into consideration, the scope of substances having the effect of "selectively activating the proliferation and/or differentiation of melanin-containing keratinocytes in the skin" cannot be specified. Therefore, claim 1 does not comply with the requirement of clearness under PCT Article 6 too.

    Such being the case, the search has been performed on the relationship between the effect of "selectively activating the proliferation and/or differentiation of melanin-containing keratinocytes in the skin" and a skin whitening method, and skin whitening methods using as the active ingredient the three kinds of plant extracts as specified in the description.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004205246 A
- JP 2004083551 A
- JP 2003245097 A
- JP 2007289063 A
- JP 6072842 A
- JP 6145038 A
- JP 2006045084 A
- JP 2004051544 A
- JP 2001302454 A
- JP 2003277251 A
- JP 2005106745 A

### Non-patent literature cited in the description

- **Cario-Andre M ; Lepreux S ; Pain C et al.** Perilesional vs. lesional skin changes in senile lentigo. *J Cutan Pathol.,* 2004, vol. 31, 441-7
- **Noblesse E ; Nizard C ; Cario-Andre M et al.** Skin ultrastructure in senile lentigo. *Skin Pharmacol Physiol,* 2006, vol. 19, 95-100
- **Unver N ; Freyschmidt-Paul P ; Horster S et al.** Alterations in the epidermal-dermal melanin axis and factor XIIIa melanophages in senile lentigo and ageing skin. *Br J Dermatol.,* 2006, vol. 155, 119-28